# EUROPEAN PATENT SPECIFICATION

(11) **EP 3 169 150 B1**
(45) Date of publication and mention of the grant of the patent: **26.09.2018**
(21) Application number: 15736680.8
(22) Date of filing: 26.06.2015
(51) Int. Cl.: A01J 5/007, A01K 29/00, H04L 12/18, A01K 1/12

(54) **DAIRY FARMING SYSTEM**
MILCHVIEHHALTUNGSSYSTEM
SYSTÈME D'ÉLEVAGE LAITIER

(30) Priority: 15.07.2014 NL 2013188
(43) Date of publication of application: 24.05.2017
(73) Proprietor: Lely Patent N.V., 3147 PB Maassluis (NL)
(72) Inventor: LOOSVELD, Serge Louis, NL-3147 PB Maassluis (NL)
(74) Representative: Octrooibureau Van der Lely N.V.
(86) International application number: PCT/NL2015/050470
(87) International publication number: WO 2016/010418

(56) References cited:
- WO-A2-2010/071413
- US-A1- 2003 146 834

## Description

The invention relates to a dairy farming system for use with a livestock building environment for dairy animals and comprising at least one dairy animals-related device which is configured to carry out a dairy animal-related action, in particular on one or more of the dairy animals, and a controller to control the dairy farming system and generate information messages, and with a communication system comprising a first communication device which is at least configured to send the information messages, and a second communication device which is at least configured to receive the information messages that have been sent by the first communication device and make them cognizable to a person.

Dairy farming systems of this type are known per se. Examples include milking robots and other automata which, in the event of a fault or the like, send an alarm message to a receiver of an operator who, as a result, does not always have to be present. On receiving an alarm or other message, the operator can then take action, if desired.

Due to advances in automation and increases in scale, it is becoming increasingly common for tasks and machines to be completely or partially automated. In practice, the efficiency of a system of this type, wherein information such as alarms is forwarded to operators or other persons, turns out not always to be optimal. This may have the result that the forwarded information is not processed, is not processed adequately or is processed too late, which can have disadvantageous consequences for the dairy animals and/or parts of the dairy farming system. Since dairy stock are livestock, this can rapidly lead to a deterioration in the health of the animals, and therefore also indirectly in human health, for example because the food supply is threatened. This is obviously undesirable.

WO2010/071413 relates to a dairy farming system according to the preamble of claim 1.

An object of the present invention is therefore to provide an improved dairy farming system which at least offers the possibility to handle the information messages more efficiently.

For this purpose, the invention provides a dairy farming system according to claim 1, in particular a dairy farming system for use in a livestock building environment for dairy animals and comprising at least one dairy animals-related device which is configured to carry out a dairy animals-related action, in particular on one or more of the dairy animals, and a controller to control the dairy farming system and generate information messages, and with a communication system comprising a first communication device which is at least configured to send the information messages, and a second communication device which is at least configured to receive the information messages that have been sent by the first communication device and make them cognizable to a person, wherein the dairy farming system is configured to define a parameter value of a parameter relating to the condition of the person, and wherein the controller is configured to send the information message and/or make it cognizable to the person depending on the defined parameter value.

Here, the invention makes use of the insight that the person who receives and has to process the message is not always readily able to do so. There may be many different reasons and causes for the inability of the person, as will be explained in detail below, but the present system can take account of this in the manner in which the information message is transferred to the receiving person, so that it can at least be better guaranteed that the information arrives and can be processed in an optimum manner. It is important to note that the present invention does not relate to settings with which, or a system wherein, the user himself actively sets his communication device to "do not disturb", or forwards it to voicemail, a different number or the like. For example, the user thus runs the risk instead that alarm messages or the like are received unnecessarily late because the "do not disturb" setting is cancelled too late. An active operation on the part of the user is also required for both setting and cancelling/modifying the setting, which is not always desirable. In the present invention, on the other hand, the dairy farming system itself is configured to determine how the information message will be transferred, i.e. depending on the parameter defined by the system. It is again noted that this does not mean that the system only establishes that the user/receiver has set his communication device to "do not disturb", "forward" or the like, but that the system decides, on the basis of a parameter that is not to be actively forwarded by the user/receiver, how to send the information message. In addition, it is obviously still an option for the controller to comprise a "do not disturb" mode, wherein information messages are either not received (and, for example, a message such as a spoken message is returned), or are held and only later communicated. In an embodiment of this type it is also clear that the present invention is more than a "do not disturb" mode given that, even if the "do not disturb" mode is not activated, information messages can be held, delayed or the like, but now depending on this defined parameter value. It should be noted that it may furthermore be possible for the second mode of the controller to be deactivatable by the user, so that in this condition the controller, i.e. the livestock farming system, operates as a system from the prior art, apart from this switchability.

The communication system comprises a first communication device for sending information and a second communication device for receiving the information. Obviously, it is also possible, but not necessary, for communication to take place from the second to the first communication device. The second communication device can be physically connected here to other parts of the controller, in particular the first communication device, such as with a wire. Examples of second communication devices of this type are, for example, a computer, such as a management computer, or a fixed telephone to which a spoken SMS message or the like can be sent. However, it is preferably a communication device that is wirelessly connected or connectable to the first communication device. Further details of this aspect will be given below.

In the present invention, the dairy farming system further comprises a milking shed environment with at least one dairy animals-related device for carrying out a dairy animals-related action, in particular on one or more of the dairy animals. In the present invention, a dairy animals-related action is understood to mean an action that is carried out in a milking shed environment on or for (non-human) dairy animals. This is understood to mean, in particular, an action performed on the body of the dairy animals, such as milking the dairy animals, or an action related thereto such as teat cleaning or after-treatment, feeding, brushing or cooling, or otherwise treating the dairy animals. This also includes actions relating to animal products which are performed in or nearby the livestock shed environment for the dairy animals, such as treating and/or moving and/or formulating feed, moving or removing manure or bedding material, and the like.

Advantageously, at least one device is an autonomous device, such as a milking robot, since it is the intention, particularly with such an autonomous device, that it is not continually supervised. For this reason, it may occur relatively more frequently that a person responsible for supervising the dairy farming system, or at least the autonomous device, is occupied with another activity or task, or is otherwise unable to process the information message in an optimum manner. The at least one device may, however, also be a different autonomous device, such as an autonomous feed pusher such as the Lely Juno™, or a non-autonomous device or machine, such as a conventional milking machine/milking carousel, feed pusher, feed-mixing device, etc. The latter machines may not operate fully autonomously, for instance the conventional milking machine/milking carousel requires the milking cups to be connected by hand, but they are able to perform another part of the operation automatically, in this case the milking itself. It is therefore possible for the device to be equated, at least part of the time, with a (fully) autonomous device, so that the same advantages are achieved with the invention.

In embodiments, the controller is configured to send the information message from the first communication device to the second communication device and to make cognizable the information message on the second communication device to the person in a first mode for non-delayed communication to the second communication device if the defined parameter value meets a first criterion, and to send the message from the first communication device to the second communication device and/or make said message cognizable to the person on the second communication device in a second, different mode if, and in particular as long as, the defined parameter value meets a second, different criterion. In the first mode, the information message is thus forwarded as quickly as possible to the person, both in terms of sending the information message from the first to the second communication device and in terms of communicating the information message to the person on the second communication device, such as with a readable or audible signal or message. In fact, the forwarding therefore relates to forwarding to the second communication device, but it is assumed here that the second communication device is actually located with the intended person, so that the person receives the information. Otherwise the advantage of the invention is at any rate barely appreciable. Here, "as quickly as possible" means that no intentional delays are built in, unlike those imposed by the prior art, such as transfer rate and speed of internal processing of the information. Intentional delay means, for example, the collection of messages by the communication system or the controller as a whole in order to forward these messages once per fixed period, such as an hour. In the first mode, the information message is thus delivered to the intended person as quickly as possible. This will be advantageous if the defined parameter value meets the first criterion, which is chosen in such a way that the likelihood is great that the receiving person is readily able to process the information message in an optimum manner.

If the defined parameter value does not meet the first criterion and if, and in particular as long as, the second criterion, which is, for example, not exclusively complementary to the first criterion, is met, the controller will send the information message from the first communication device to the second communication device and/or make said message cognizable to the person on the second communication device, in the second, different mode. The controller switches over to this second mode if it appears from the defined parameter value that the likelihood is great, or at any rate sufficiently greater, that the receiving person cannot process the information message efficiently enough. The dairy farming system can thus take account of the fact that the receiver cannot respond in an optimum manner. How and under what conditions the dairy farming system, and, in particular, the controller, respond will be explained in detail below.

In embodiments, the first criterion comprises that the defined parameter value has a predefined value or lies in a predefined interval, and the second criterion comprises that the parameter value does not have the predefined value or lies outside that interval, in particular lies in a second interval. By means of a parameter of this type, the dairy farming system can then make a distinction in order to choose the correct mode. It should be noted that the number of modes could also be extended to three or more, with a corresponding number of criteria. The parameter value can relate here to a physical value, but also a relative or even abstract value, such as "person is busy", though a value of this type will in most cases be determined on the basis of one or more physical values Furthermore, the interval may be unlimited on one or even both sides. In this last case, the first criterion requires that the parameter value lies outside a limited interval. However, the interval will often be limited on both sides. The interval may relate to a real interval, such as with physical values, or may also comprise natural or whole number values or a collection of abstract values.

In embodiments, the controller is configured in the second mode to delay sending the information message from the first communication device or communicating the information message to the person until either the defined parameter value meets the first criterion, or until a third criterion is met. In this way, it can be sufficiently guaranteed that the information message is processed efficiently. The third criterion may, for example, comprises that one or more of the defined parameter values improves to a predefined extent, i.e the exceeding of a limit value decreases relatively or absolutely to a predefined extent.

In embodiments, the controller is configured in the second mode to send the message to a different, third communication device. Thus, if it is likely that the information message will be uncertainly processed by the receiving person, the controller can choose to send the information message to an alternative person, i.e. a third communication device. It should be noted that this does not include the parameter or situation wherein a telephone call or the like is forwarded if a telephone line or other communication line is busy. It involves a parameter of the person, not the terminal itself.

In embodiments, the controller is configured to send information messages with different priority, such as pure information, alert signals and alarm signals, and the controller is configured to send the information messages from the first communication device to the second communication device or to make cognizable said messages on the second communication device depending on the priority. In estimating whether and how efficiently the information can be processed, the controller takes account of the possible impact of the information message. Thus, with expected moderate attention, a purely informative message will probably be disregarded, but an alarm will actually be processed. It may then be appropriate to defer the sending of the purely informative message until the sending of an alarm, since concentration and attention will possibly have improved thereafter. Obviously, some monitoring can also be carried out by means of the parameter values to be defined.

In embodiments, the parameter is a physical condition parameter of the person, and the dairy farming system comprises a sensor actively connected to the controller to measure the parameter value of the person. This sensor, or one of the sensors, is, for example, provided close to the controller or the dairy animals-related device, but, in particular, on the second communication device so that the sensor is usually close to the intended person, i.e. the carrier of the second communication device. A physical condition parameter is chosen here as the parameter. These parameters can provide a clear estimation relating to the concentration of the person, or the extent to which incoming information messages can be efficiently processed. However, measurement of different or additional parameters is not excluded. The sensor is actively connected to the controller, such as directly communicating with the controller, or to the second communication device or otherwise. For example, the sensor forms part of the second communication device, but that is not obligatory. It may, for example, comprise a camera with image recognition which is configured to determine a concentration level, indicatively or relatively or otherwise, from the physical characteristics, such as the posture of the head and body, facial expression, speed of movement, etc. Systems of this type are known per se, for example from the automotive sector. The camera can be provided on the tablet or laptop computer or smartphone, but also in a livestock building or office. The camera may be person-tracking.

It is furthermore noted here that the controller may be self-learning, wherein the controller is configured to adjust at least the first criterion on the basis of responses to information messages that have been made cognizable to the person in the past. If, in the case of a specific parameter value, for example a given information message that required timely or even immediate action was nevertheless disregarded, the controller can conclude that the measured parameter value still fell in an interval wherein the concentration of the user was insufficient. The edges of the interval for the first criterion can then be actively adjusted accordingly by the controller.

In embodiments, the parameter comprises a stress level parameter, in particular an acceleration, a heart rate, a blood pressure and/or a respiration frequency, and the sensor comprises an accelerometer, a heart rate monitor, a blood pressure monitor and/or a respiration frequency monitor. Alternative or additional parameters are operating speed and/or precision and/or force of the second communication device, and the sensor comprises a keystroke speed monitor and/or keystroke precision monitor and/or keystroke force monitor. Particularly in the case of modern operating interfaces with a touch screen, this is an attractive design. A high stress level is a clear indication that the receiving person is less able to process the information message correctly and/or in a timely manner. A stress level of this type can be measured in many ways, and, in particular, by means of the indicated sensors. For example, a heart rate monitor can be provided which measures the heart rate of the person. If or as long as the heart rate is above a defined limit value according to a first criterion, the controller can for example, delay sending the message. The accelerometer measures whether the person is subject to accelerations. This may in fact be an indication of agitated movements or otherwise, such as running, driving or the performance of all manner of actions. Situations of this type are indicative of a reduced concentration level in relation to incoming information messages. It is noted here that the accelerometer, if installed in the second communication device, actually measures the accelerations of this second communication device, but it is assumed here that the accelerations of the person are comparable. Furthermore, it is noted that the aforementioned sensors may also be provided separately on the person, such as a heart rate monitor (of the type known from fitness aids), a blood pressure or respiration frequency monitor. Alternatives are certainly conceivable, such as on the basis of facial recognition or a direction of view sensor, of the type already known per se from the automotive industry for detecting drowsiness of a driver. A stress level can also be derived from the manner of operation. If the number of keystrokes or the keystroke force is higher than an absolute threshold value, or, for example, a relative threshold value linked to an average, which may be a running average or otherwise, for the person operating the second communication device, this can be a clear indication of an increased stress level. The controller can then decide to switch over to the second mode.

In embodiments, the parameter value comprises an ambient parameter value of the person, in particular a sound pressure level around the person or a position of the person. This parameter value may be an alternative or additional indication of the concentration of the person. The person will thus be able to receive, register and/or process the information less well in a noisy environment. The sensor to be used is then, in particular, a noise pressure meter. Depending on the position, it may also be difficult for the person to process the information. For example, an information message may be difficult to process in a part of a domestic environment, such as a bathroom. Similarly, it may be difficult or more difficult to process the information if the person is not located in or in the vicinity of the livestock building environment, such as abroad, on the water, in a forest, shop or the like. Some details may, for example, be recorded in the form of GPS coordinates. An area can then be selected wherein, if the receiver, i.e. the second communication device, is located in that area, the second mode is chosen by the controller. Other configurations are also possible.

In embodiments, the controller is configured to determine the parameter value as "busy" if the person is busy with one of a predefined group of activities comprising the operation of a component of the dairy farming system, in particular the second communication device, or a device in an information exchange connection with the dairy farming system, in particular the second communication device, and "not busy" if that is not the case. The "busy" parameter value, or a technical equivalent thereof, relates to situations in which it is establishable that the receiving person is busy with an activity that requires his attention. This can be established, for example, by determining whether the person is operating the second communication device, such as by operating buttons or entering information. Alternatively or additionally, it may be establishable that the person is busy with a different activity, such as, in particular, the operation of a device directly or indirectly connected to the second communication device, such as a different device of the dairy farming system, particularly a milking machine or robot, feed device, feed management computer, etc. For this purpose, the controller can receive operating signals and allocate the "busy" or "not busy" parameter value on the basis thereof. Depending on this parameter value, the information message can then be sent, i.e. directly and undelayed, or delayed, or to a different receiver, etc. A clear example of this relates to the operation of a computer as the second communication device. The computer is then, in particular, a management computer which is intended and configured to manage the dairy farming system. This may relate to a desktop computer or laptop computer, but also variants thereof such as a laptop computer or tablet computer, or even a smartphone with one or more "apps". If the second communication device establishes that the person is entering information, or is processing other information or otherwise using the communication device, such as photographing, phoning or listening to music, the controller can choose the second mode.

In embodiments, the dairy animals-related device comprises a milking device, in particular a milking robot or a milking carousel, or a spraying robot or an autonomous livestock building vehicle, such as a manure slide or feed pusher. Such devices, which are often complicated and deal with variable and largely unpredictable livestock, may send a multiplicity of information messages, which sometimes require a low-level of attention to interpret, but sometimes also require a high-level of attention. For instance, it might be the case with a milking carousel comprising conventional milking devices that the milking cups are attached by hand, but that a dairy animal kicks away the cups out of the direct sight of the person connecting the cups. The information message which is then required may not be interpreted in some circumstances, or may be incorrectly interpreted, so that it is possible to optimize the sending thereof in accordance with the invention. The effect of the invention can be seen even more clearly in completely autonomous dairy animals-related devices, as these operate, in principle, without direct human supervision, and an information message must therefore always be sent in the case of a fault, which message must also be processed in an optimum manner as some time will pass before the supervisor reaches the device.

The communication system is not particularly restricted. In particular, the communication system comprises Bluetooth®, ZigBee®, Wi-Fi, a mobile telephone connection or Internet connection. By means of communication systems of this type, an efficient information transfer is possible under many circumstances and there is also a wide variety of possibilities in the manner of transfer. For example, mobile telephone connections and also Internet connections have an extensive coverage and, due to the many available extras, such as sensors and applications ("apps"), they are very suitable for use in the present invention.

In embodiments, the second communication device comprises a so-called "wearable", such as a mobile telephone, a PDA, a laptop, a smart watch, smart glasses or a tablet computer. Second communication devices of this type are simply wearable on the person. In addition, more and more devices of this type are already provided as standard with one or more sensors that are useful in the context of the present invention, such as a camera, an accelerometer, etc., so that this sensor can be used with a simple software program (or "app") to determine one or more parameter values. Obviously, it is also possible to provide one or more dedicated sensors on the device, or to provide an alternative communication device, such as a pager or even a camera with communication means.

The invention will be explained below with reference to the drawing, wherein the single figure shows a schematic view of an embodiment of a dairy farming system according to the invention.

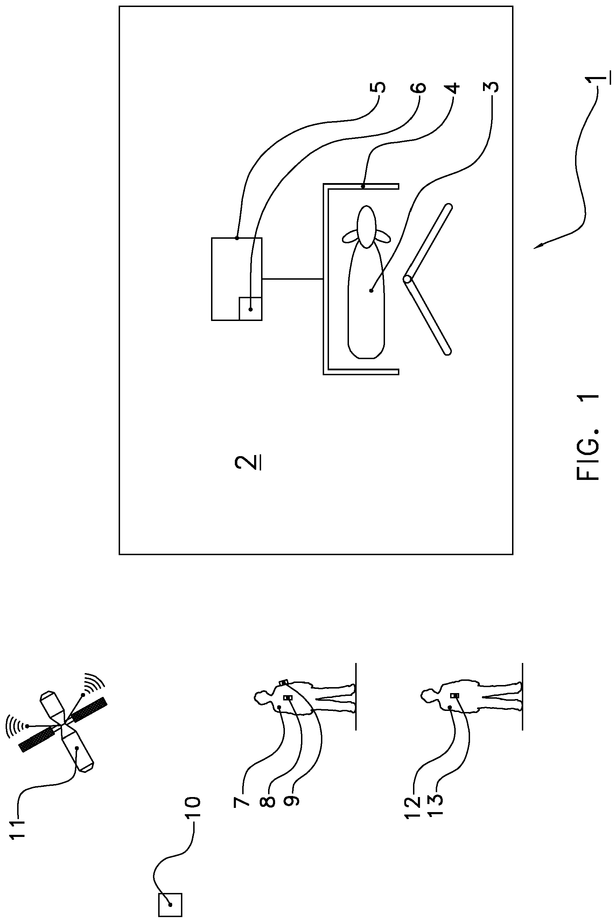

Figure 1 shows a schematic view of a dairy farming system 1 according to the invention.

The dairy farming system 1 is provided in a milking shed environment 2 for cows 3, and comprises a milking robot 4 controlled by a controller 5 having a first communication device 6.

A first person carrying a second communication device 8 and a heart rate monitor 9 is denoted by 7. A sound pressure meter is denoted by 10 and a communication satellite by 11.

A second person 12 carries a third communication device 13. The dairy farming system 1 thus comprises a communication system which in turn comprises at least the communication devices 6 and 8, and optionally also the third communication device 13.

The dairy farming system 1 comprises a milking shed environment 2 for dairy animals, such as dairy cows 3. In this case, the shed environment 2 is adapted to the type of dairy animal accommodated therein, and comprises one or more dairy animals-related devices. In this case, for example, a milking robot 4 is provided which is configured to autonomously milk the cows 3. A controller 5 is provided for this purpose.

If a milking operation of the cow 3 fails, the milk is contaminated, the cow 3 displays a symptom of mastitis or there is a mechanical or other fault, etc., the controller 5 will attempt to send an information message to a supervising first person 7, via the first and second communication devices 6 and 8, for example a GSM transmitter or a mobile telephone. The communication satellite 11 can be used here. Alternatively, 6 is a Bluetooth® or Wi-Fi transmitter, and 8 is a Bluetooth® or Wi-Fi receiver, or a different communication system, and the satellite 11 is not necessary.

The aforementioned message may differ in type and priority. Thus, a fault in the milking robot 4 which prevents further milking or even threatens the health of the animal to be milked at that time will have a much higher priority than a mastitis symptom of a cow 3. It may thus be of importance to ensure as well as possible that the first person 7 who receives the information message processes the information well and takes any necessary action.

All manner of circumstances may influence that information-processing and action-taking capability, such as an increased stress level and a high level of ambient noise. In order to be able to take account of this, the system 1 here comprises, for example, a heart rate monitor 9 and a sound pressure meter 10 which are actively connected to the controller, either directly or via at least the second communication device. If, for example, the heart rate monitor measures a heart rate of more than 100 beats per minute, the system 1, in particular the second communication device 8 or controller 5, concludes that the first person 7 is not readily able to process the information to be sent, or information possibly already sent but not yet made cognizable. Alternatively or additionally, if the sound pressure level as measured by the sound pressure meter 10 is above 70 dB(A), the system, in particular the second communication device 8 or controller 5, concludes that the first person 7 is not readily able to process the information to be sent. Other values for the parameters are obviously possible, as well as other parameters, which then require associated sensors. The posture of the body and head has already been mentioned above.

It is also possible to establish whether the first person 7 is "busy", since the system 1 looks at the operation of the second communication device 8 or possibly the milking robot 4. In this last case, it may be advantageous to link the position of the first person 7, as to be determined, for example, by means of GPS coordinates of the second communication device 8, to the position of the milking robot. If the first person 7 is "busy", the controller can similarly establish that the first person is not readily able to process the information message well and quickly.

If the system 1 has established that the first person 7 is not readily able to process the information to be sent or sent, it may decide to delay the sending or communicating (such as showing on a telephone screen) until the parameter values lie within a permitted range. This may apply, for example, to non-emergency information messages. For emergency messages, such as alarms and faults, the system 1 can alternatively choose to send the information message to an alternative, in this case a second person 12, who carries a third communication device 13, such as a mobile telephone or tablet computer.

The embodiment shown is not intended to be limiting, but only to clarify the invention. The scope of protection is determined on the basis of the attached claims.

## Claims

1. A dairy farming system (1) for use in a livestock building environment (2) for dairy animals (3) and comprising:
- at least one dairy animals-related device (4) which is configured to carry out a dairy animals-related action, in particular on one or more of the dairy animals, and
- a controller (5) to control the dairy farming system and generate information messages, and with a communication system comprising a first communication device (6) which is at least configured to send the information messages, and a second communication device (8) which is at least configured to receive the information messages that have been sent by the first communication device and make them cognizable to a person (7), **characterised in that** the dairy farming system is configured to define a parameter value of a parameter relating to the condition of the person, and wherein the controller is configured to send the information message and/or make it cognizable to the person depending on the defined parameter value.

2. The dairy farming system as claimed in claim 1, wherein the controller is configured to send the information message from the first communication device to the second communication device and to make cognizable the information message on the second communication device to the person in a first mode for non-delayed communication to the second communication device if the defined parameter value meets a first criterion, and to send the message from the first communication device to the second communication device and/or make said message cognizable to the person on the second communication device in a second, different mode if, and in particular as long as, the defined parameter value meets a second, different criterion.

3. The dairy farming system as claimed in claim 2, wherein the first criterion comprises that the defined parameter value has a predefined value or lies in a predefined interval, and the second criterion comprises that the parameter value does not have the predefined value or lies outside that interval, in particular lies in a second interval.

4. The dairy farming system as claimed in claim 2 or 3, wherein the controller is configured in the second mode to delay sending the information message from the first communication device or communicating the information message to the person until either the defined parameter value meets the first criterion, or until a third criterion is met.

5. The dairy farming system as claimed in one of claims 2-4, wherein the controller is configured in the second mode to send the message to a different, third communication device (13).

6. The dairy farming system as claimed in one of the preceding claims, wherein the controller is configured to send information messages with different priority, such as pure information, alert signals and alarm signals, and the controller is configured to send the information messages from the first communication device to the second communication device or to make cognizable said messages on the second communication device depending on the priority.

7. The dairy farming system as claimed in one of the preceding claims, wherein the parameter is a physical condition parameter of the person, and the dairy farming system comprises a sensor (9, 10) actively connected to the controller to measure the parameter value of the person.

8. The dairy farming system as claimed in claim 7, wherein the parameter comprises a stress level parameter, in particular an acceleration, a heart rate, a blood pressure and/or a respiration frequency, and the sensor comprises an accelerometer, a heart rate monitor (9), a blood pressure monitor and/or a respiration frequency monitor.

9. The dairy farming system as claimed in claim 7 or 8, wherein the parameter value comprises an ambient parameter value of the person, in particular a sound pressure level around the person or a position of the person, and wherein the sensor comprises a sound pressure meter (10).

10. The dairy farming system as claimed in any preceding claim, wherein the controller is configured to determine the parameter value as "busy" if the person is busy with one of a predefined group of activities comprising the operation of a component of the dairy farming system, in particular the second communication device, or a device in an information exchange connection with the dairy farming system, in particular the second communication device, and "not busy" if that is not the case.

11. The dairy farming system as claimed in any one of the preceding claims, wherein the dairy animals-related device comprises a milking robot (4), a milking carousel, a spraying robot or an autonomous livestock building vehicle, such as a manure slide or feed pusher.

12. The dairy farming system as claimed in one of the preceding claims, wherein the communication system comprises Bluetooth®, ZigBee®, a mobile telephone connection or Internet connection.

13. The dairy farming system as claimed in one of the preceding claims, wherein the second communication device is a wearable, such as a mobile telephone (8, 13), a PDA, a laptop, a smart watch, smart glasses or a tablet computer.

## Patentansprüche

1. Milchviehhaltungssystem (1) zur Verwendung in einer Stallumgebung (2) für Milchtiere (3), das Folgendes umfasst:
- wenigstens eine milchtierbezogene Vorrichtung (4), die konfiguriert ist, eine milchtierbezogene Handlung, insbesondere an einem oder mehreren der Milchtiere, auszuführen und
- eine Steuerung (5), um das Milchviehhaltungssystem zu steuern und Informationsnachrichten zu erzeugen, und mit einem Kommunikationssystem, das eine erste Kommunikationsvorrichtung (6), die konfiguriert ist, wenigstens die Informationsnachrichten zu senden, und eine zweite Kommunikationsvorrichtung (8), die konfiguriert ist, wenigstens die Informationsnachrichten, die von der ersten Kommunikationsvorrichtung gesendet worden sind, zu empfangen und diese für eine Person (7) erkennbar zu machen, umfasst,
**dadurch gekennzeichnet, dass**
das Milchviehhaltungssystem konfiguriert ist, einen Parameterwert eines Parameters zu definieren, der sich auf den Zustand der Person bezieht, wobei die Steuerung konfiguriert ist, in Abhängigkeit von dem definierten Parameterwert die Informationsnachricht zu senden und/oder diese für die Person erkennbar zu machen.

2. Milchviehhaltungssystem nach Anspruch 1, wobei die Steuerung konfiguriert ist, die Informationsnachricht von der ersten Kommunikationsvorrichtung zu der zweiten Kommunikationsvorrichtung zu senden und die Informationsnachricht auf der zweiten Kommunikationsvorrichtung für die Person in einer ersten Betriebsart für eine nicht verzögerte Kommunikation zu der zweiten Kommunikationsvorrichtung erkennbar zu machen, falls der definierte Parameterwert ein erstes Kriterium erfüllt, und die Nachricht von der ersten Kommunikationsvorrichtung zu der zweiten Kommunikationsvorrichtung zu senden und/oder die Nachricht für die Person auf der zweiten Kommunikationsvorrichtung in einer zweiten, anderen Betriebsart erkennbar zu machen, falls der definierte Parameterwert ein zweites, anderes Kriterium erfüllt, und solange er dieses erfüllt.

3. Milchviehhaltungssystem nach Anspruch 2, wobei das erste Kriterium umfasst, dass der definierte Parameterwert einen zuvor definierten Wert aufweist oder innerhalb eines zuvor definierten Intervalls liegt, und wobei das zweite Kriterium umfasst, dass der Parameterwert den zuvor definierten Wert nicht aufweist oder außerhalb des Intervalls liegt, insbesondere innerhalb eines zweiten Intervalls liegt.

4. Milchviehhaltungssystem nach Anspruch 2 oder 3, wobei die Steuerung konfiguriert ist, in der zweiten Betriebsart das Senden der Informationsnachricht von der ersten Kommunikationsvorrichtung oder die Kommunikation der Informationsnachricht zu der Person zu verzögern, bis der definierte Parameterwert das erste Kriterium erfüllt oder bis ein drittes Kriterium erfüllt ist.

5. Milchviehhaltungssystem nach einem der Ansprüche 2-4, wobei die Steuerung in der zweiten Betriebsart konfiguriert ist, die Nachricht zu einer anderen, dritten Kommunikationsvorrichtung (13) zu senden.

6. Milchviehhaltungssystem nach einem der vorhergehenden Ansprüche, wobei die Steuerung konfiguriert ist, die Informationsnachrichten, etwa reine Informationen, Warnsignale und Alarmsignale mit unterschiedlicher Priorität zu senden, und wobei die Steuerung konfiguriert ist, die Informationsnachrichten von der ersten Kommunikationsvorrichtung zu der zweiten Kommunikationsvorrichtung zu senden oder die Nachrichten auf der zweiten Kommunikationsvorrichtung abhängig von der Priorität erkennbar zu machen.

7. Milchviehhaltungssystem nach einem der vorhergehenden Ansprüche, wobei der Parameter ein Parameter eines physischen Zustands der Person ist und wobei das Milchviehhaltungssystem einen Sensor (9, 10) umfasst, der mit der Steuerung aktiv verbunden ist, um den Parameterwert der Person zu messen.

8. Milchviehhaltungssystem nach Anspruch 7, wobei der Parameter einen Stressgradparameter, insbesondere eine Beschleunigung, eine Herzfrequenz, einen Blutdruck und/oder eine Atemfrequenz umfasst, und wobei der Sensor einen Beschleunigungsmesser, einen Herzfrequenzmonitor (9), einen Blutdruckmonitor und/oder einen Atemfrequenzmonitor umfasst.

9. Milchviehhaltungssystem nach Anspruch 7 oder 8, wobei der Parameterwert einen Umgebungsparameterwert der Person, insbesondere einen Schalldruckpegel um die Person oder eine Position der Person umfasst und wobei der Sensor einen Schalldruckmesser (10) umfasst.

10. Milchviehhaltungssystem nach einem der vorhergehenden Ansprüche, wobei die Steuerung konfiguriert ist, den Parameterwert als "beschäftigt" festzulegen, falls die Person mit einer Aktivität aus einer vordefinierten Gruppe von Aktivitäten beschäftigt ist, die das Betreiben einer Komponente des Milchviehhaltungssystems, insbesondere der zweiten Kommunikationsvorrichtung oder einer Vorrichtung in einer Informationsaustauschverbindung mit dem Milchviehhaltungssystem, insbesondere der zweiten Kommunikationsvorrichtung umfasst, und als "nicht beschäftigt" festzulegen, falls dies nicht der Fall ist.

11. Milchviehhaltungssystem nach einem der vorhergehenden Ansprüche, wobei die milchtierbezogene Vorrichtung einen Melkroboter (4), ein Melkkarussell, einen Sprühroboter oder ein autonomes Stallfahrzeug, wie etwa eine Dungrutsche oder ein Futterschieber, umfasst.

12. Milchviehhaltungssystem nach einem der vorhergehenden Ansprüche, wobei das Kommunikationssystem Bluetooth®, ZigBee®, eine Mobiltelefonverbindung oder eine Internetverbindung umfasst.

13. Milchviehhaltungssystem nach einem der vorhergehenden Ansprüche, wobei die zweite Kommunikationsvorrichtung eine tragbare Vorrichtung wie etwa ein Mobiltelefon (8, 13), ein PDA, ein Laptop, eine Smartwatch, Smartglasses oder ein Tablet-Computer ist.

## Revendications

1. Système d'élevage laitier (1) destiné à être utilisé dans un environnement (2) de bâtiment d'élevage pour des animaux laitiers (3) et comprenant :
- au moins un dispositif (4) associé aux animaux laitiers configuré pour exécuter une action associée aux animaux laitiers, en particulier sur un ou plusieurs des animaux laitiers, et
- une unité de commande (5) pour commander le système d'élevage laitier et générer des messages d'information, et avec un système de communication comprenant un premier dispositif de communication (6) qui est au moins configuré pour envoyer les messages d'information, et un deuxième dispositif de communication (8) qui est au moins configuré pour recevoir les messages d'information qui ont été envoyés par le premier dispositif de communication et les porter à la connaissance d'une personne (7),
**caractérisé en ce que**
le système d'élevage laitier est configuré pour définir une valeur paramétrique d'un paramètre se rapportant à l'état de la personne, et dans lequel l'unité de commande est configurée pour envoyer le message d'information et/ou le porter à la connaissance de la personne en fonction de la valeur paramétrique définie.

2. Système d'élevage laitier selon la revendication 1, dans lequel l'unité de commande est configurée pour envoyer le message d'information du premier dispositif de communication au deuxième dispositif de communication et porter le message d'information se trouvant sur le deuxième dispositif de communication à la connaissance de la personne dans un premier mode pour une communication non retardée au deuxième dispositif de communication si la valeur paramétrique définie satisfait un premier critère, et envoyer le message du premier dispositif de communication au deuxième dispositif de communication et/ou porter ledit message se trouvant sur le deuxième dispositif de communication à la connaissance de la personne dans un second mode différent, si, et en particulier tant que, la valeur paramétrique définit satisfait un deuxième critère différent.

3. Système d'élevage laitier selon la revendication 2, dans lequel le premier critère est que la valeur paramétrique définie a une valeur prédéfinie ou est comprise dans un intervalle prédéfini, et le deuxième critère est que la valeur paramétrique n'a pas la valeur prédéfinie ou n'est pas comprise dans cet intervalle, en particulier est comprise dans un second intervalle.

4. Système d'élevage laitier selon la revendication 2 ou 3, dans lequel l'unité de commande est configurée dans le second mode pour retarder l'envoi du message d'information du premier dispositif de communication ou communiquer le message d'information à la personne jusqu'à ce que soit la valeur paramétrique définie satisfasse le premier critère, soit un troisième critère soit satisfait.

5. Système d'élevage laitier selon l'une des revendications 2 à 4, dans lequel l'unité de commande est configurée dans le second mode pour envoyer le message à un troisième dispositif de communication différent (13).

6. Système d'élevage laitier selon l'une des revendications précédentes, dans lequel l'unité de commande est configurée pour envoyer des messages d'information de priorités différentes, tels que des informations uniquement, des signaux d'avertissement et des signaux d'alarme, et l'unité de commande est configurée pour envoyer les messages d'information du premier dispositif de communication au deuxième dispositif de communication ou pour rendre perceptible lesdits messages se trouvant sur le deuxième dispositif de communication en fonction de la priorité.

7. Système d'élevage laitier selon l'une des revendications précédentes, dans lequel le paramètre est un paramètre d'état physique de la personne, et le système d'élevage laitier comprend un capteur (9, 10) connecté activement à l'unité de commande pour mesurer la valeur paramétrique de la personne.

8. Système d'élevage laitier selon la revendication 7, dans lequel le paramètre le paramètre comprend un paramètre de niveau de stress, en particulier une accélération, un rythme cardiaque, une pression artérielle et/ou une fréquence de respiration, et le capteur comprend un accéléromètre, un moniteur de rythme cardiaque (9), un moniteur de pression artérielle et/ou un moniteur de fréquence de respiration.

9. Système d'élevage laitier selon la revendication 7 ou 8, dans lequel la valeur paramétrique comprend une valeur paramétrique ambiante de la personne, en particulier un niveau de pression acoustique autour de la personne ou d'une position de la personne, et dans lequel le capteur comprend un compteur de pression acoustique (10).

10. Système d'élevage laitier selon l'une quelconque des revendications précédentes, dans lequel l'unité de commande est configurée pour déterminer la valeur paramétrique comme étant "occupée" si la personne est occupée dans le cadre d'un groupe prédéfini d'activités comprenant le fonctionnement d'un composant du système d'élevage laitier, en particulier du deuxième dispositif de communication, ou d'un dispositif dans une connexion d'échange d'informations avec le système d'élevage laitier, en particulier le deuxième dispositif de communication, et "non occupée" dans le cas contraire.

11. Système d'élevage laitier selon l'une quelconque des revendications précédentes, dans lequel le dispositif associé aux animaux laitiers comprend un robot de traite (4), un carrousel de traite, un robot de pulvérisation ou un véhicule de bâtiment d'élevage de bétail autonome, tel qu'un poussoir d'évacuation ou d'alimentation de lisier.

12. Système d'élevage laitier selon l'une des revendications précédentes, dans lequel le système de communication comprend une connexion Bluetooth®, une connexion ZigBee®, une connexion de téléphone portable ou une connexion Internet.

13. Système d'élevage laitier selon l'une des revendications précédentes, dans lequel le deuxième dispositif de communication est un dispositif portable sur soi, tel qu'un téléphone portable (8, 13), un PDA, un ordinateur portable, une montre intelligente, des lunettes intelligentes ou une tablette informatique.
